# EUROPÄISCHE PATENTANMELDUNG

(11) **EP 3 476 387 A1**
(43) Veröffentlichungstag der Anmeldung: **01.05.2019**
(21) Anmeldenummer: 17199045.0
(22) Anmeldetag: 27.10.2017
(51) Int. Cl.: A61K 9/14, A23K 20/20, A23K 50/20, A23K 50/10, A23K 50/75, A23K 50/30, A23K 50/40, A23L 33/16

(54) **VERFAHREN ZUM HERSTELLEN VON ZINKOXID ZUR ALIMENTÄREN VERWENDUNG BEI SCHWEINEN, ANDEREN MONOGASTRISCHEN TIEREN, PFERDEN ODER WIEDERKÄUERN, ZINKOXID ZUR ALIMENTÄREN VERWENDUNG BEI SCHWEINEN, ANDEREN MONOGASTRISCHEN TIEREN, PFERDEN ODER WIEDERKÄUERN, UND ZINKOXID ZUR VERWENDUNG IN EINEM PROPHYLAKTISCHEN ODER THERAPEUTISCHEN VERFAHREN BEI SCHWEINEN ODER ANDEREN MONOGASTRISCHEN TIEREN**

(71) Anmelder: ISF GmbH, 23812 Wahlstedt (DE)
(72) Erfinder: Mathies, Edmund, 25436 Moorrege (DE); Ramhold, Dietmar, 23820 Pronstorf (DE); Rimbach, Martin, 24558 Henstedt-Ulzburg (DE); Strauch, Wolfram, 22587 Hamburg (DE)
(74) Vertreter: Hauck Patentanwaltspartnerschaft mbB

(57) **Zusammenfassung**

Verfahren zum Herstellen von Zinkoxid zur alimentären Verwendung bei Schweinen, anderen monogastrischen Tieren, Pferden oder Wiederkäuern, bei dem Zinkoxid im festen Aggregatzustand auf einen mittleren Durchmesser der Partikel von maximal 50 |am vermahlen und zugleich mechanisch aktiviert wird.

## Beschreibung

Die Erfindung betrifft ein Verfahren zum Herstellen von Zinkoxid geeignet zur alimentären Verwendung bei Schweinen, anderen monogastrischen Tieren, Pferden oder Wiederkäuern, ein Zinkoxid geeignet zur alimentären Verwendung bei Schweinen, anderen monogastrischen Tieren, Pferden oder Wiederkäuern und ein Zinkoxid zur Verwendung in einem prophylaktischen oder therapeutischen Verfahren bei Schweinen oder anderen monogastrischen Tieren.

Spurenelemente - Zink, Kupfer, Mangan, Selen, Kobalt, Eisen, Jod etc. - sind lebensnotwendige Nährstoffe für alle Tiere. Sie haben viele Funktionen im Körper und sind somit einzeln oder gemeinsam im allgemeinen Stoffwechsel, der Reproduktion, dem Immunsystem, dem Wachstum, der Entwicklung und Regenerierung verschiedener Gewebe usw. beteiligt.

Zink ist wichtiger Bestandteil vieler (über 300) Enzyme, die an der Synthese und dem Abbau von Kohlenhydraten, Fetten, Proteinen, Nukleinsäure und auch dem Stoffwechsel anderer Mikronährstoffe beteiligt sind. Zink stabilisiert die molekulare Struktur von zellulären Bestandteilen und Membranen und trägt auf diese Weise zur Erhaltung der Zell- und Organtätigkeit bei. Des Weiteren spielt Zink eine wichtige Rolle in der Transkription und somit Genexpression. Durch seine Beteiligung an diesen fundamentalen Prozessen ist Zink essentiell für alle Lebensformen. Ferner hat Zink eine zentrale Funktion im Immunsystem, da es sowohl das zelluläre als auch das humorale Immunsystem beeinflusst.

Beim Schwein kann eine Unterversorgung mit Zink Haut-, Haar- und Klauenschäden, Skelettschäden, Fruchtbarkeitsstörungen und eine verminderte Immunität zur Folge haben. Zur Verbesserung der Leistung sowie des Wachstums wird Zink in Form von Zinkoxid (ZnO) im Futter supplementiert. Bei langfristiger Überversorgung mit mehr als 1000 mg/kg Futter kann es allerdings zur Minderleistung kommen.

Ferner ist Zinkoxid in vielen Ländern der EU ein weitverbreiteter pharmazeutischer Zusatz im Futter von früh abgesetzten Ferkeln, um Durchfälle zu vermeiden. Hier wird Zinkoxid in therapeutischen Dosierungen weit über den futterrechtlichen Grenzwerten (3.000 vs. 150 ppm) für maximal 14 Tage nach dem Absetzen eingesetzt, wenn es durch einen Tierarzt verschrieben wurde. Hingegen liegt in einigen Ländern, wie z. B. auch der BRD, ein Verbot für solche Anwendungen vor. Durch den erheblichen Umwelteinfluss hoher ZnO-Dosierungen wird dieser Anwendungsbereich aber sehr kritisch in der EU betrachtet.

Zinkoxidpulver wird als Standardware für die Zulage zum Futtermittel in der Qualität "*Feed Grade*" beispielsweise von der Firma Grillo kommerzialisiert. Der größte Gewichtsmengenanteil an dem weltweit produzierten Zinkoxid wird durch ein Brennverfahren hergestellt.

Um eine Anreicherung von Zink in Ackerböden durch ausgebrachte Schweinegülle zu vermeiden, wurde eine drastische Senkung der Spurenelementezulage zum Futter diskutiert. Unter anderem wurde angeregt, zu untersuchen, ob durch besser verfügbare Spurenelementverbindungen, z. B. organisch gebundene Spurenelemente, eine höhere Sicherheit bei der Versorgung der Schweine auf abgesenktem Niveau erreicht werden kann. Ferner wurde die Untersuchung der Wirkung eingesetzter Phytasen auf die Verfügbarkeit der Spurenelemente vorgeschlagen (Gerhard Stalljohann, Andrea Meyer: "Weniger Kupfer und Zink ins Schweinefutter?", Topagrar 2/2003, Seiten 17-19).

Für die leistungsverbessernde und das Risiko für Absetzdurchfälle von Ferkeln reduzierende Zinkoxid-Supplementierung wird von der Firma Animine unter der Produktbezeichnung "HiZox®" ein spezielles Zinkoxid vermarktet, dass die negativen Auswirkungen eines überhöhten und anhaltenden Einsatzes von Zinkoxid auf Tiere und Umwelt vermeiden soll. Nach Angaben des Anbieters handelt es sich hierbei um ein sogenanntes potenziertes Zinkoxid, das aufgrund eines speziellen Herstellungsverfahrens eine gegenüber herkömmlichem Zinkoxid ca. 15-fach vergrößerte Oberfläche aufweist. Hierdurch soll die Kontaktfläche gegenüber Bakterien drastisch zunehmen. Dabei soll die spezifische poröse Struktur des Granulats die antibakterielle Aktivität dieses potenzierten Zinkoxids verstärken (Agathe Roméo, Valérie Kromm: "Antibakterielle Effekte von Zinkoxid bei Absetzferkeln", European Feed Business Magazin 9-10, 2016, Seiten 1-5).

Bei HiZox® wurde gegenüber Standardware eine verstärkte Löslichkeit des Zinks in Wasser festgestellt und die verstärkte antibakterielle Aktivität auf eine erhöhte "Bioverfügbarkeit" der gelösten Zinkionen zurückgeführt (W. Vahjen, J. Zentek, S. Durosoy: "Inhibitory action of two zink oxide sources on the ex-vivo growth of porcine small intestine bacteria", J. Anim Sci. 2012.90; 334 bis 336).

Davon ausgehend liegt der Erfindung die Aufgabe zugrunde, ein Zinkoxid zur Verfügung zu stellen, das die gewünschte alimentäre, prophylaktische oder therapeutische Wirkung bei Schweinen, anderen monogastrischen Tieren, Pferden oder Wiederkäuern, insbesondere die Vermeidung von Absetzdurchfällen bei Ferkeln oder die Unterstützung der Regenerierung von Hufen, Füßen und Haut bei Schweinen, Pferden oder Wiederkäuern, mit verringertem Mengeneinsatz ermöglicht.

Die Aufgabe wird durch ein Verfahren zum Herstellen eines Zinkoxids mit den Merkmalen von Anspruch 1 gelöst. Ferner wird die Aufgabe durch ein Zinkoxid mit den Merkmalen von Anspruch 14 gelöst. Schließlich wird die Aufgabe durch ein Zinkoxid mit den Merkmalen von Anspruch 15 gelöst. Vorteilhafte Ausführungsarten der Erfindung sind in Unteransprüchen angegeben.

Bei dem erfindungsgemäßen Verfahren zum Herstellen von Zinkoxid geeignet zur alimentären Verwendung bei Schweinen, anderen monogastrischen Tieren, Pferden oder Wiederkäuern, wird Zinkoxid im festen Aggregatzustand auf einen mittleren Durchmesser der Partikel von maximal 50 µm vermahlen und zugleich mechanisch aktiviert.

Das erfindungsgemäße Zinkoxid geeignet zur alimentären Verwendung bei Schweinen, anderen monogastrischen Tieren, Pferden oder Wiederkäuern, ist erhältlich durch das erfindungsgemäße Verfahren oder eine seiner Ausgestaltungen.

Das erfindungsgemäße Zinkoxid zur Verwendung in einem prophylaktischen oder therapeutischen Verfahren bei Schweinen, insbesondere zur vorbeugenden alimentären Verwendung bei durchfallgefährdeten Schweinen, insbesondere Absetzferkeln, oder bei anderen monogastrischen Tieren, ist erhältlich durch das erfindungsgemäße Verfahren oder eine seiner Ausgestaltungen.

Das erfindungsgemäße Zinkoxid (mikronisiertes Zinkoxid) hat *in vitro* und *in vivo* eine gegenüber Standardware und gegenüber potenziertem Zinkoxid verbesserte Wirkung. Im Laborversuch kommt es zu einer verstärkten Hemmung des Wachstums von E. coli gegenüber der Kontrolle (Standard-Zinkoxidpulver und potenziertes Zinkoxid) und im Tierversuch zu einer reduzierten Durchfallrate gegenüber der Kontrolle (Standard Zinkoxidpulver). Durch die Vermahlung und Aktivierung wird die Oberfläche vergrößert und eine deutlich aktivere Substanz erreicht. Die chemisch/physikalische Änderung der Aktivität durch die Vermahlung und Aktivierung ist auch an der pH-Aktivität des ansonsten inerten Zinkoxides zu erkennen. Zudem ist die Löslichkeit des Zinks in Wasser deutlich erhöht. Die deutliche Erhöhung des pH-Wertes und der höhere Anteil an gelösten Zink-Ionen beruht auf einer verstärkten Reaktion des Zinkoxids mit Wasser und damit einer insgesamt verstärkten Reaktivität des Zinkoxids. Zudem zeigt die trotz geringerer spezifischer Oberfläche erhöhte Hemmwirkung des mikronisierten Zinkoxids gegenüber dem potenzierten Zinkoxid HiZox® von Animine den verstärkenden Einfluss der Aktivierung auf die Keimhemmung.

Aufgrund der Aktivierung des erfindungsgemäßen Zinkoxids ist zu erwarten, dass sämtliche gewünschten alimentären Wirkungen mit verringertem Mengeneinsatz erreicht werden.

Die bei der alimentären Anwendung bei Ferkeln festgestellte prophylaktische Wirkung ist auch bei erwachsenen Schweinen und anderen monogastrischen Tieren (Geflügel, Katzen, Hunde etc.) zu erwarten. Bei Monogastriern handelt es sich entgegengesetzt zu Wiederkäuern um Tiere mit einem einfachen bzw. einhöhligen Magen. Wiederkäuer besitzen hingegen ein mehrhöhliges Magensystem, welches durch sein großes Volumen und das Vorhandensein von Mikroben das aufgenommene Futter fermentiert. Zu den Wiederkäuer werden z. B. Rinder, Schafe und Hirsche und zu den Monogastriern z. B. Menschen, Katzen und Hunde gezählt. Im landwirtschaftlichen Bereich stellen Schweine, Geflügel und Pferde den Hauptanteil der Monogastrier dar, die sich anatomisch aber erst im hinteren Teil des Verdauungssystems (Dickdarmbereich) gravierend voneinander unterscheiden. Alle Monogastrier müssen im Gegensatz zu Wiederkäuern mit hochwertigeren Protein- und Kohlenhydratquellen ernährt werden. Einige Spezies der Monogastrier ernähren sich ausschließlich von pflanzlicher Nahrung (Herbivoren), z. B. das Pferd. Andere wiederum sind Allesfresser (Omnivoren), die sich sowohl von pflanzlicher als auch tierischer Nahrung ernähren können. Wohingegen z. B. Katzen zu den Karnivoren gezählt werden und dementsprechend ausschließlich tierische Nahrung fressen. Beim Schwein handelt es sich um ein omnivores Tier, welches somit anatomisch und physiologisch ein gutes Modelltier für Erklärung und Interpretation von Wirkungsmechanismen beim Monogastrier im Allgemeinen darstellt.

Aufgrund der Aktivierung des erfindungsgemäßen Zinkoxids ist eine Unterstützung der Regenerierung von Hufen, Füßen und Haut bei Schweinen, Pferden und Wiederkäuern (EU-VO 5/2014) mit verringertem Mengeneinsatz des Zinkoxids zu erwarten.

Das mechanisch aktivierte Zinkoxid weist eine gegenüber dem nicht mechanisch aktivierten Zustand erhöhte freie Enthalpie auf. Die Erhöhung der freien Enthalpie beruht vor allem auf Gitterstörungen und Strukturfehlern, die den Festkörpern durch Beanspruchung mit genügend hoher Intensität zugefügt werden.

Durch die mechanische Aktivierung wird insbesondere die Reaktivität von Festkörpern erhöht. Hierzu wird Bezug genommen auf Heinrich Schubert: "Handbuch der Verfahrenstechnik", WILEY-VCH, Seite 200 ff. und Matthias Bohnet: "Mechanische Verfahrenstechnik", WILEY-VCH, Seite 160 ff.

Gemäß einer weiteren Ausführungsart wird die freie Enthalpie um mindestens ein kJ/mol erhöht. Gemäß einer weiteren Ausführungsart beträgt der spezifische Energieeintrag beim Vermahlen und mechanischen Aktivieren des Zinkoxids mindestens 100, vorzugsweise 150 bis 300 kWh/t.

Der "mittlere Durchmesser der Partikel" bzw. "mittlere Partikeldurchmesser" ist definiert als Durchmesser d₅₀. Dies bedeutet, dass 50 % der Partikelgrößenverteilung (Basis: Massenverteilung) kleiner sind als der angegebene Wert.

Gemäß einer Ausführungsart der Erfindung ist das Ausgangsprodukt für die Vermahlung und mechanische Aktivierung des Zinkoxids ein Zinkoxidpulver mit einem mittleren Durchmesser der Partikel von über 50 µm. Vorzugsweise ist das Ausgangsprodukt eine Standardware. Das Ausgangsprodukt ist beispielsweise die eingangs erwähnte Standardware der Firma Grillo. Hierzu wird Bezug genommen auf das Stoffdatenblatt zu Zinkoxid F80 (Version D-ZnO-F80/5 Date of Revision: 01/11) der Grillo Zinkoxid GmbH.

Gemäß einer weiteren Ausführungsart wird das Zinkoxid in einer Mühle für die Feinstmahlung auf einen mittleren Partikeldurchmesser im Bereich von 5-50 µm, Mikrofeinmahlung auf einen mittleren Partikeldurchmesser im Bereich von 0,5-5 µm oder Nanofeinmahnung auf einen mittleren Partikeldurchmesser kleiner als 0,5 µm vermahlen und mechanisch aktiviert.

Gemäß einer weiteren Ausführungsart wird das Zinkoxid mittels einer Kugelmühle, Schwingmühle, Rührwerkmühle, Planetenmühle oder einer anderen Mahlkörpermühle vermahlen und mechanisch aktiviert.

Gemäß einer bevorzugten Ausführungsart wird das Zinkoxid mittels einer Schwingmühle mit Unwuchtantrieb (z. B. Exzenter-Schwingmühle) vermahlen und mechanisch aktiviert.

Gemäß einer weiteren Ausführungsart wird das Zinkoxid in einer Mühle mittels Prall- und Stoßbeanspruchung sowie Reibbeanspruchung vermahlen und mechanisch aktiviert. Die Prall- und Stoßbeanspruchung kann zwischen Mahlgutpartikeln, Mahlkörpern und Wandung der Mühle und die Reibbeanspruchung zwischen den Mahlgutpartikeln im Gutbett untereinander und durch die umlaufenden Mahlkörper erfolgen. Eine Exzenter-Schwingmühle kombiniert die vorstehenden Beanspruchungsmechanismen.

Gemäß einer bevorzugten Ausführungsart wird das Zinkoxid im trockenen Zustand vermahlen und mechanisch aktiviert.

Gemäß einer weiteren Ausführungsart wird das Zinkoxid auf einen mittleren Partikeldurchmesser von maximal 30 µm, vorzugsweise von maximal 20 µm, vorzugsweise von maximal 15 µm, vorzugsweise von maximal 10 µm vermahlen. Gemäß einer weiteren Ausführungsart ist das Zinkoxid auf einen mittleren Partikeldurchmesser von mindestens 2 µm, vorzugsweise von mindestens 5 µm vermahlen.

Gemäß einer weiteren Ausführungsart wird das Zinkoxid so vermahlen und mechanisch aktiviert, dass seine spezifische Oberfläche mindestens 0,2 m²/g, vorzugsweise mindestens 0,25 m²/g beträgt. Gemäß einer weiteren Ausführungsart wird das Zinkoxid so vermahlen und mechanisch aktiviert, dass eine spezifische Oberfläche maximal 0,5 m²/g, vorzugsweise maximal 0,45 m²/g beträgt.

Gemäß einer weiteren Ausführungsart wird das Zinkoxid während einer Mahldauer von mindestens 10 Minuten, vorzugsweise von mindestens 20 Minuten, vorzugsweise von mindestens 30 Minuten vermahlen und mechanisch aktiviert. Gemäß einer bevorzugten Ausführungsart erfolgt hierbei die Vermahlung und mechanische Aktivierung in einer Schwingmühle.

Gemäß einer weiteren Ausführungsart wird das Zinkoxid so vermahlen und mechanisch aktiviert, dass die Sättigungskonzentration der Zinkionen im Wasser mindestens vier mg/l, vorzugsweise mindestens 5 mg/l beträgt.

Gemäß einer weiteren Ausführungsart wird das Zinkoxid so vermahlen und mechanisch aktiviert, dass der pH-Wert nach Zugabe von 10 g Zinkoxid zu 100 ml destilliertem Wasser, intensivem Schütteln über eine Stunde und Ruhenlassen über 30 Minuten im flüssigen Überstand mindestens 8,6, vorzugsweise mindestens 8,8 beträgt.

Ferner betrifft die Erfindung ein Futtermittel zur alimentären, prophylaktischen oder therapeutischen Verwendung bei Schweinen, insbesondere zur vorbeugenden alimentären Verwendung bei durchfallgefährdeten Schweinen, insbesondere Absetzferkeln, enthaltend maximal 3.000 ppm, vorzugsweise maximal 2.000 ppm, vorzugsweise maximal 1.000 ppm, vorzugsweise maximal 160 ppm, vorzugsweise maximal 120 ppm, vorzugsweise maximal 100 ppm, vorzugsweise maximal 65 ppm Zinkoxid, erhältlich durch das Verfahren nach einem der Ansprüche 1 bis 13. Der futtermittelrechtlich zulässige Höchstgehalt an Zink beträgt in der EU für Ferkel, Sauen, Kaninchen und alle Fischarten außer Salmoniden 150 ppm in Alleinfuttermitteln (Basis - 88 % Trockenmasse). Unter Berücksichtigung eines nativen Zinkgehaltes von 30 ppm im Alleinfuttermittel (Basis - 88 % Trockenmasse) dürfen dann maximal 160 ppm Zinkoxid dem Alleinfuttermittel (Basis - 88 % Trockenmasse) zugelegt werden.

Ferner betrifft die Erfindung einen Futterzusatzstoff oder Futtermittelzusatzstoff enthaltend, bestehend im Wesentlichen aus oder bestehend aus Zinkoxid, erhältlich durch ein Verfahren nach einem der Ansprüche 1 bis 13.

Ferner betrifft die Erfindung ein Ergänzungsfuttermittel zur Unterstützung der Regenerierung von Hufen, Füßen und Haut bei Schweinen, Pferden oder Wiederkäuern, das Zinkoxid enthält, erhältlich durch das Verfahren nach einem der Ansprüche 1 bis 13. Dabei handelt es sich um ein Futtermittel für besondere Ernährungszwecke im Sinne der EU-VO 5/2014. Das Ergänzungsfuttermittel darf Zink in einer Konzentration von mehr als dem 100-fachen des entsprechend festgelegten Höchstgehalts in Alleinfuttermitteln enthalten.

Ferner betrifft die Erfindung die Verwendung von Zinkoxid erhältlich durch das Verfahren nach einem der Ansprüche 1 bis 13 bei der Ernährung von Schweinen, Pferden und Wiederkäuern zur Unterstützung der Regenerierung von Hufen, Füßen und Haut. Hierbei handelt es sich um eine Verwendung im Sinne der EU-VO 5/2014. Gemäß einer bevorzugten Ausführungsart erfolgt die Ernährung durch Gabe eines das Zinkoxid enthaltenden Ergänzungsfuttermittels. Alternativ kann die Gabe durch Einmischung des Zinkoxids in das Trinkwasser erfolgen.

Ferner betrifft die Erfindung ein Zinkoxid gemäß Anspruch 13 oder 15, ein Futtermittel gemäß Anspruch 16, einen Futterzusatzstoff oder Futtermittelzusatzstoff obiger Art (Seite 10, Abs. 1), ein Ergänzungsfuttermittel gemäß Anspruch 17 oder die Verwendung eines Zinkoxids gemäß Anspruch 18, wobei das Zinkoxid statt durch die Herstellbarkeit durch das Verfahren nach einem der Ansprüche 1 bis 13 dadurch gekennzeichnet ist, dass es mechanisch aktiviert ist, einen mittleren Partikeldurchmesser gemäß Anspruch 1, 7 oder 8 und eine spezifische Oberfläche gemäß Anspruch 9 oder 10 aufweist. Gemäß einer weiteren Ausführungsart ist die freie Enthalpie des Zinkoxids um mindestens 1 kJ/mol gegenüber dem nicht aktivierten Zustand erhöht. Gemäß einer weiteren Ausführungsart ist das Zinkoxid so beschaffen, dass die Zinkionen in Wasser eine Sättigungskonzentration gemäß Anspruch 12 aufweisen. Gemäß einer weiteren Ausführungsart ist das Zinkoxid so beschaffen, dass es den pH-Wert von destilliertem Wasser gemäß Anspruch 13 erhöht.

Die Erfindung wird nachfolgend anhand der Ergebnisse von Vergleichsuntersuchungen näher erläutert.

Gemäß dem nachfolgenden Versuchsbericht "Vergleich verschiedener Zinkoxid-Produkte in einem Mikrotiterplattensystem" bewirkt das erfindungsgemäße Zinkoxid (ZnOmicro) eine deutliche Hemmung des Wachstums von E. coli JM 109 bei geringeren Grenzkonzentrationen als Standardware und aufbereitetes Zinkoxidpulver eines Mitbewerbers.

### 1. Beschreibung und Ziel

Es soll die Hemmwirkung verschiedener Zinkoxid-Produkte gegen einen ausgewählten Testorganismus in einem Mikrotitersystem geprüft werden.
- ZnO (Zinkoxid Pulver feed grade, Standardware Fa. Grillo)
- ZnOmicro (Zinkoxid Pulver mikronisiert in einer Exzenterschwingmühle, ISF GmbH)
- ZnO_V (Zinkoxid Pulver aufbereitet, Vergleichsprodukt eines Mitbewerbers)

| | |
|---|---|
| Testorganismus: | *Escherichia coli* JM 109 |
| Methode: | Hemmtest in Mikrotiterplatten (MTP) |

### 2. Methode zur Bestimmung

### der Hemmwirkung auf Mikrotiterplatten (MTP)

### 2.1 Prinzip der Methode:

Ausgewählte Verdünnungsstufen von ZnO, ZnOmicro und ZnO_V werden auf MTP in Nährbouillon (NB) vorgelegt (je 100µl). Als negative Kontrolle werden in zwei Reihen jeweils 100 µl NB aufgetragen. In einem zweiten Schritt wird der Teststamm (je 100 µl von *E. coli* JM109) aufgebracht.

Die Platten werden bei 37 °C inkubiert und nach 0 h, 6 h und 24 h wird das Wachstum des Teststammes über die Messung der Trübung (OD₆₀₀) im MTP-Reader (TECAN Genios) ermittelt.

### 2.2 Durchführung:

### Vorbereitung der MTP-Platte mit den Testsubstanzen:

Die verschiedenen Zinkoxid-Produkte ZnO, ZnOmicro und Zn_V werden in steriler NB gelöst bzw. suspendiert und auf eine Konzentration von 8000 mg/l standardisiert. Im Mikrotiterplattensystem werden durch Verdünnung mit steriler NB in Zweierstufen die Konzentrationen: 4000 / 2000 / 1000 / 500 / 250 / 125 / 62,5 / 31,3 / 15,6 / 7,8 mg/l hergestellt.

### Vorkultur des Teststammes:

50 ml NB werden mit 200 µl des bei -80 °C tiefgefrorenen Teststammes *E. coli* JM109 beimpft und über Nacht im Schüttler bei 130 rpm und 37 °C bebrütet. Die Zellsuspension wird durch Verdünnen in steriler NB auf eine OD₆₀₀ von 0,2 standardisiert.

### Durchführung des Tests:

Je 100 µl der standardisierten Zellsuspension werden in die einzelnen Wells mit 100 µl der vorgelegten Testsubstanz in den verschiedenen Verdünnungen bzw. der Negativkontrolle pipettiert.

### Inkubation und Auswertung:

Die MTP werden bei 37 °C aerob inkubiert, nach 0 h, 6 h und 24 h wird das Wachstum des Teststammes über die Messung der Trübung im MTP-Reader (OD₆₀₀) ermittelt.

Der 0 h - Wert wird als Blindwert jeweils von den späteren Messwerten abgezogen.

### 3 Ergebnisse und Schlussfolgerung

### 3.1 Ergebnisse aus dem Hemmtest im MTP-System

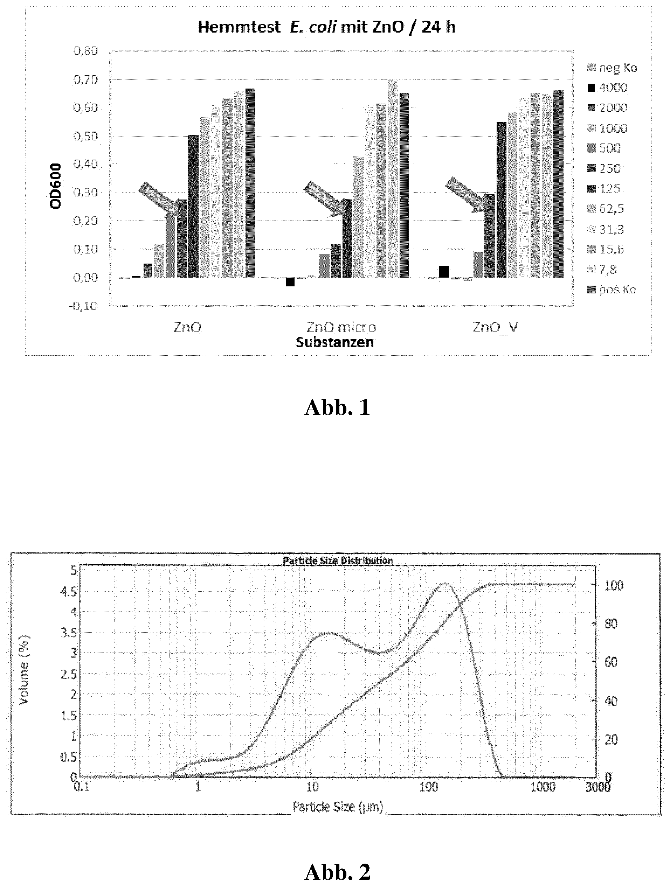

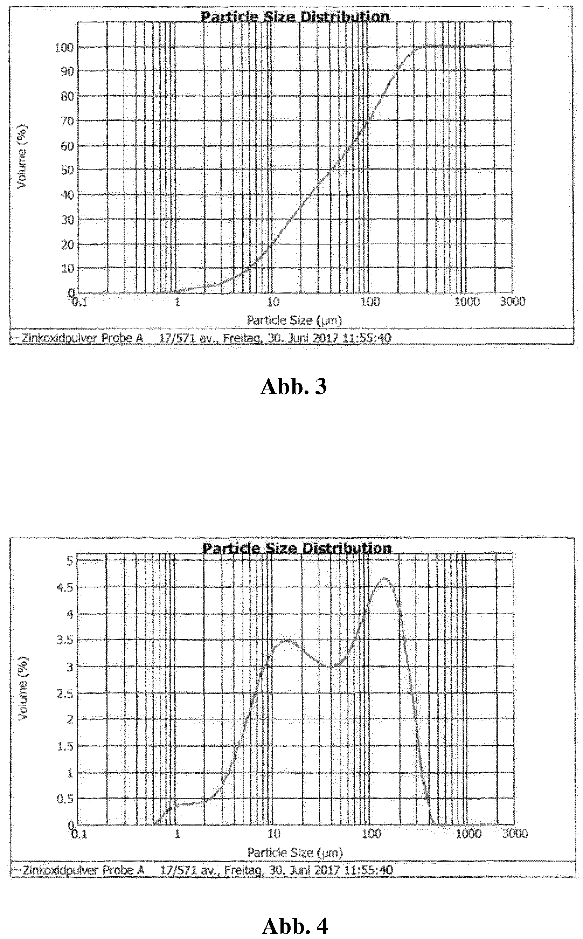

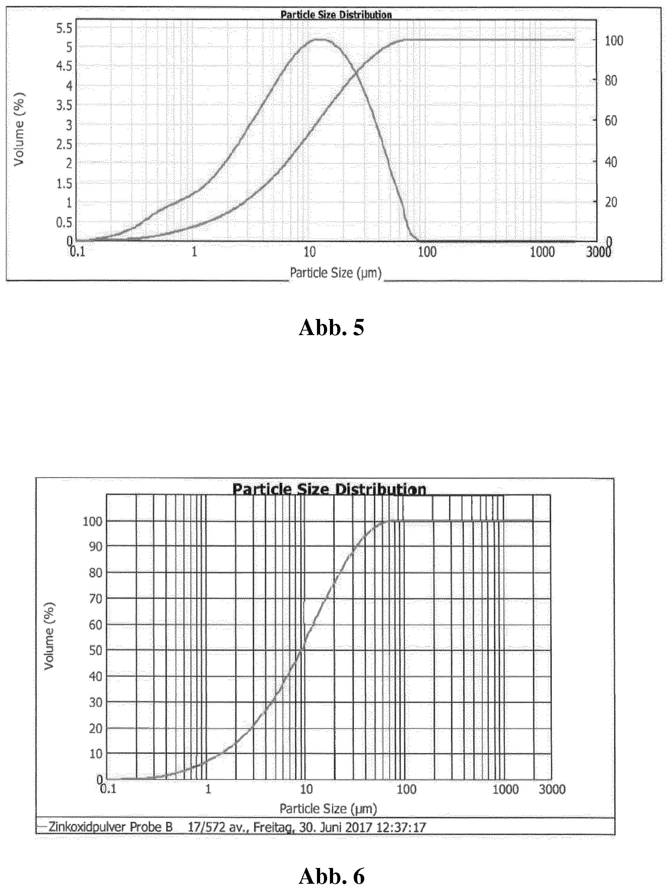

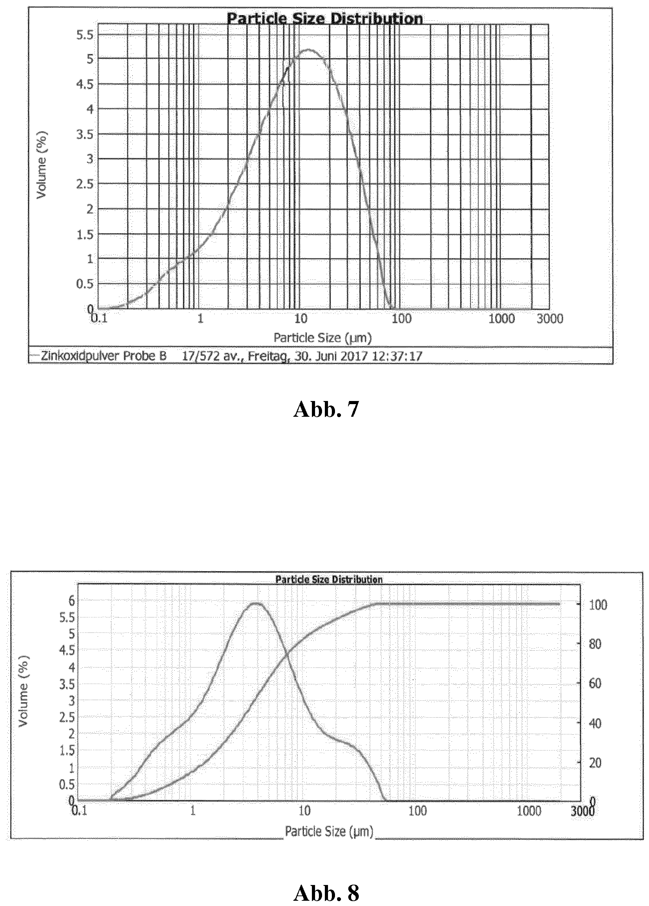

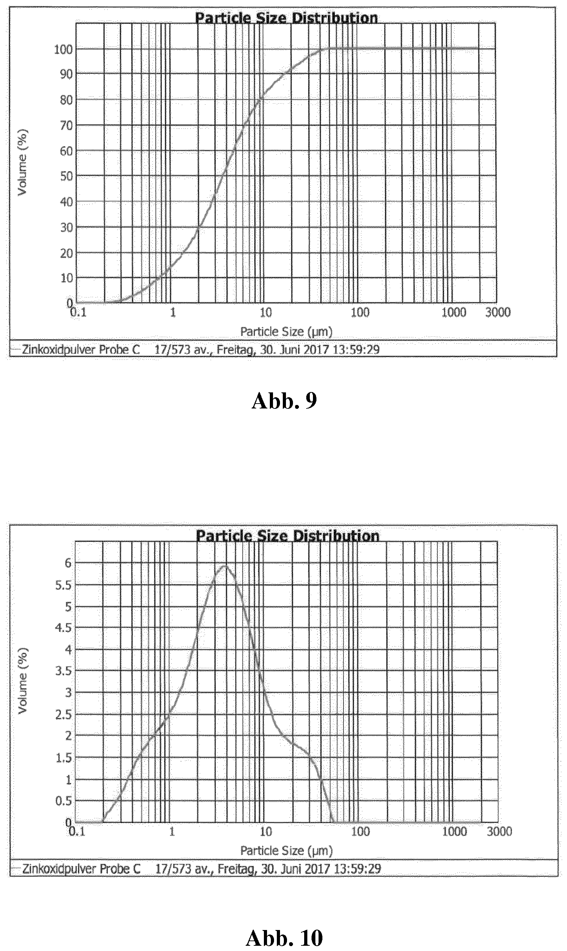

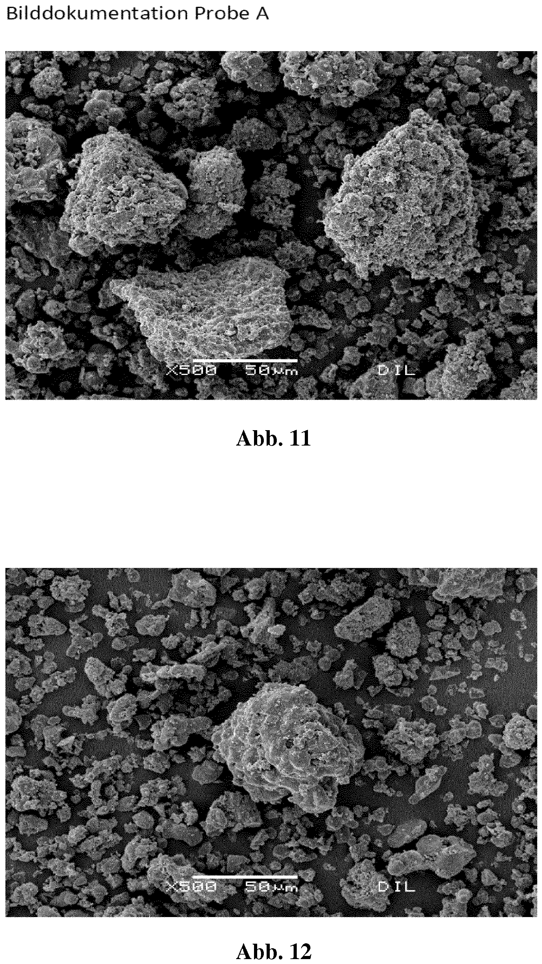

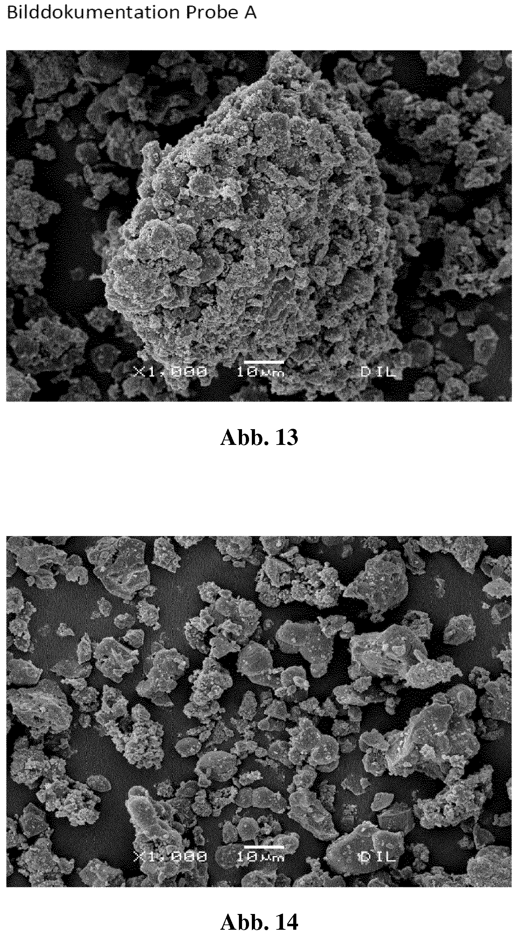

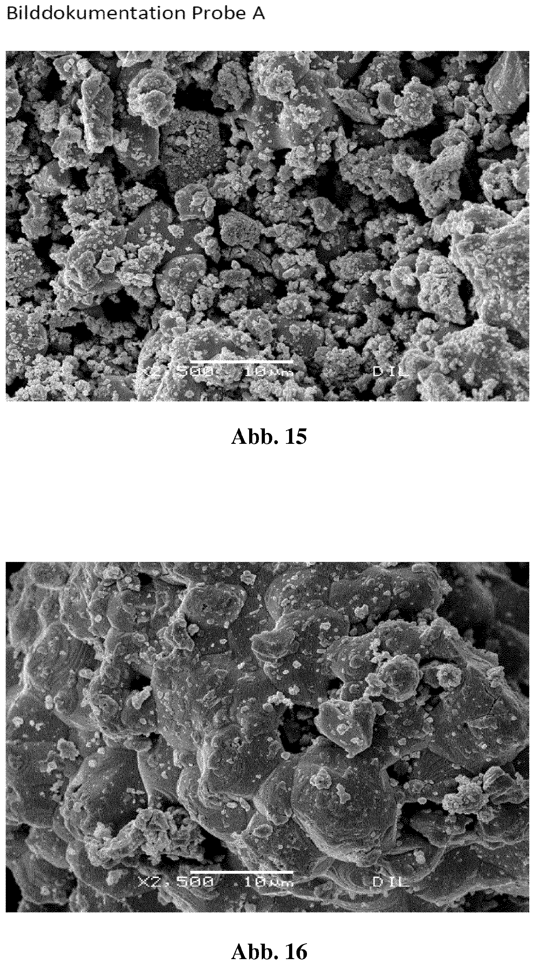

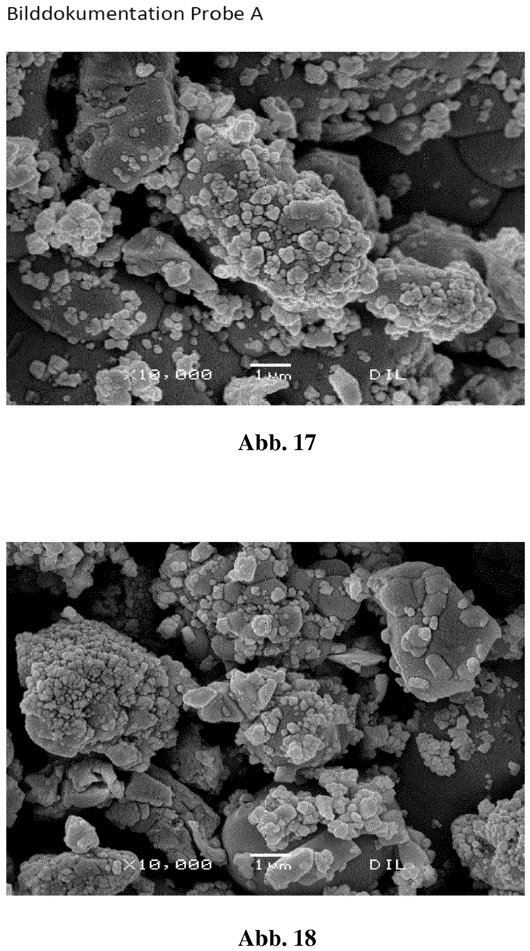

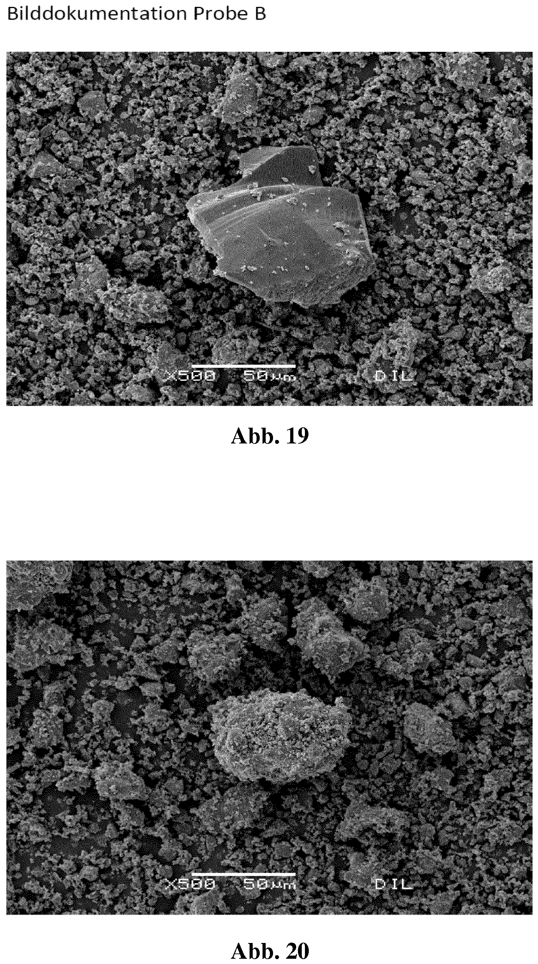

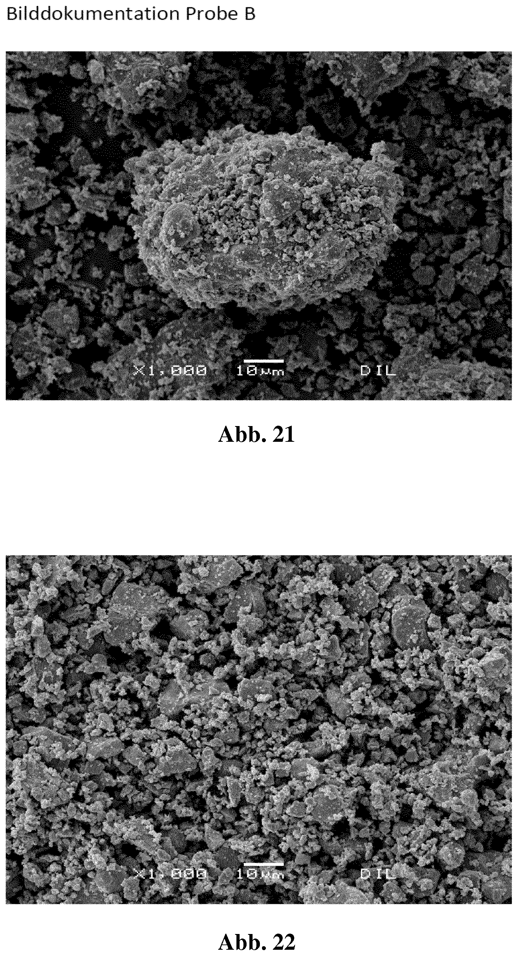

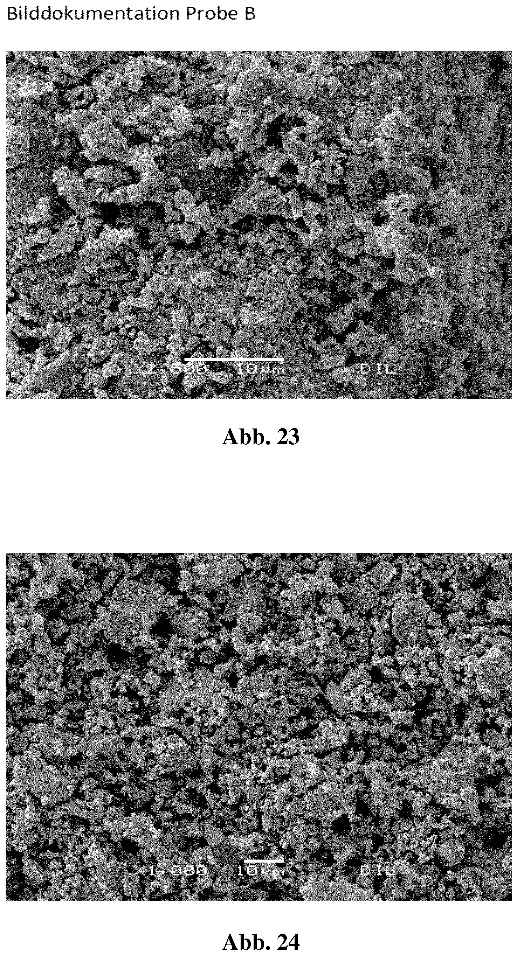

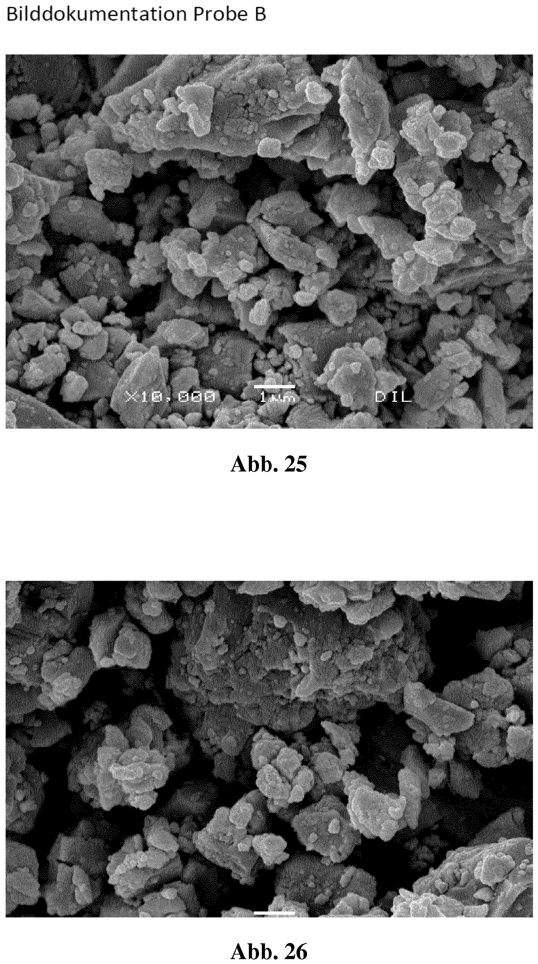

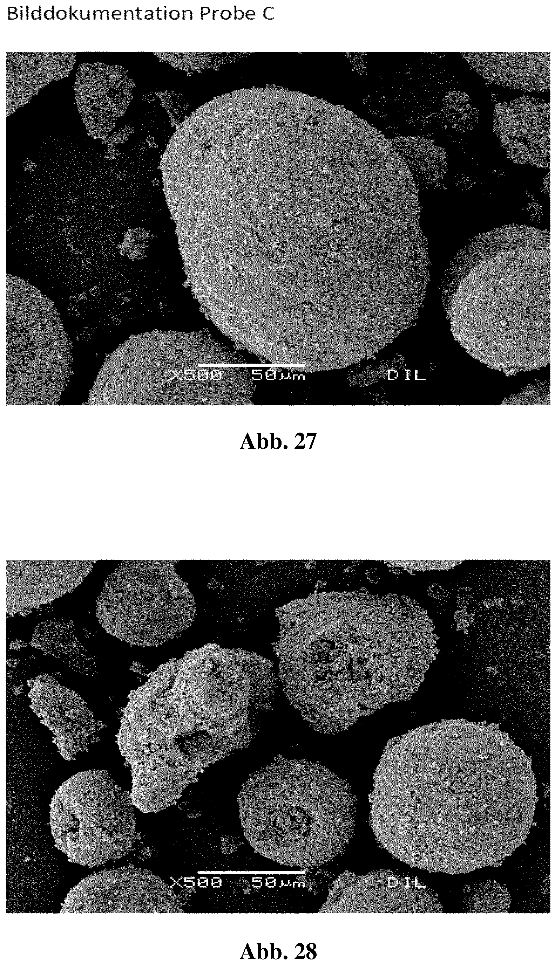

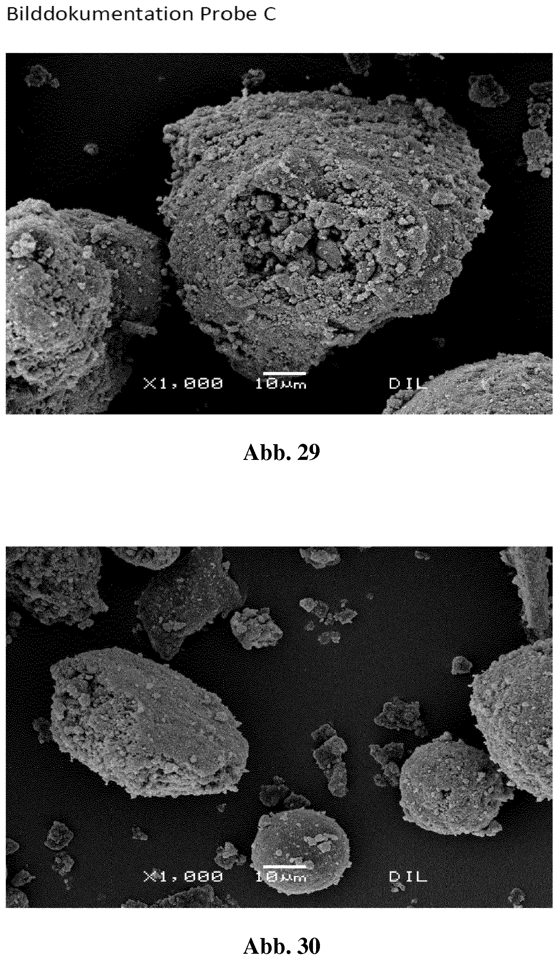

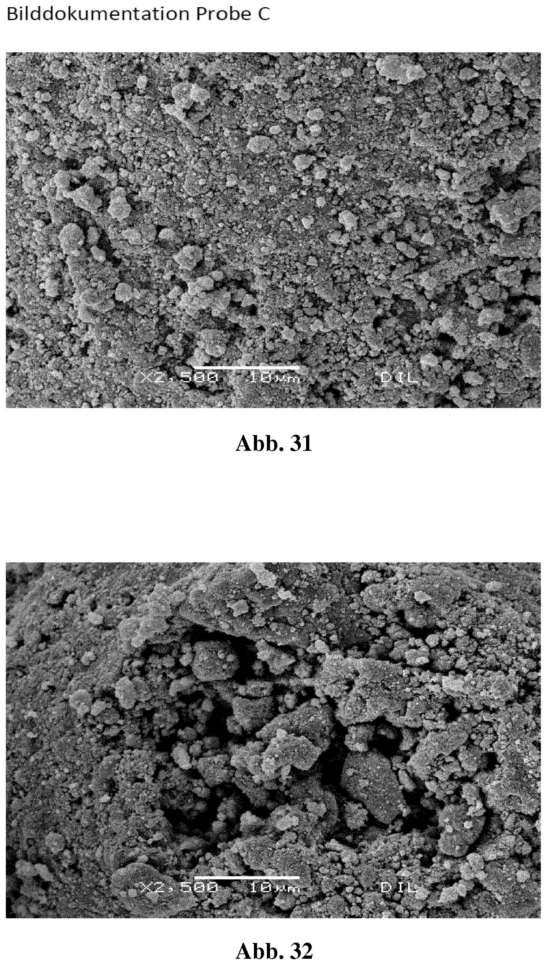

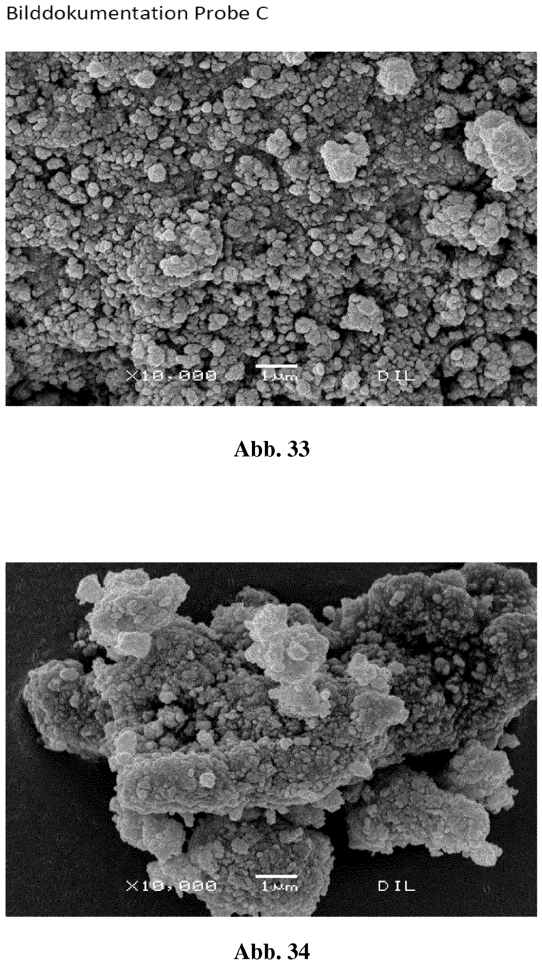

Das Wachstum von *E. coli* JM109 wurde durch die Messung der optischen Dichte bei 600 nm (OD₆₀₀) ermittelt. Eine Reduktion des OD₆₀₀-Wertes nach 24 h um 50 % im Vergleich zur Kontrolle wird als deutliche Hemmung des Wachstums gewertet und die zugehörige Konzentration der Testsubstanz als Grenzkonzentration ermittelt. Die grünen Pfeile in Abb. 1 markieren die Grenzkonzentrationen der einzelnen Testsubstanzen.

In der unten angeführten Tabelle 1 sind die Grenzkonzentrationen für eine deutliche Hemmung des Wachstums des Teststammes *E. coli* (JM109) durch die verschiedenen Zinkoxid-Produkte nochmals zusammengefasst.

**Tabelle 1: Grenzkonzentration für eine deutliche Hemmung des Wachstums von E. coli JM109 (mg/l)**

| | Grenzkonzentration (mg/l) |
|---|---|
| ZnO | 250 |
| ZnOmicro | 125 |
| ZnO_V | 250 |

### 4. Schlussfolgerung:

Der Teststamm E. coli JM109 wird durch das mikronisierte und aktivierte Zinkoxid der Fa. ISF GmbH am besten gehemmt. Bei einer Konzentration von 125 mg/l wird das Wachstum des Teststammes im verwendeten Testsystem um mehr als 50 % im Vergleich zu einer Negativkontrolle reduziert.

Eine vergleichbare Hemmung wird durch die Zinkoxid Standardware ZnO (Zinkoxid Pulver feed grade der Fa. Grillo) und ein aufbereitetes Zinkoxid Mitbewerberprodukt (ZnO-V) erst bei einer Konzentration von 250 mg/l erreicht.

Gemäß der nachfolgenden Untersuchung "Charakterisierung von Zinkoxidpulver" hat das erfindungsgemäße Zinkoxidpulver (Probe B) eine größere spezifische Oberfläche als die Standardware (Probe A) und eine geringere spezifische Oberfläche als aufbereitetes Zinkoxidpulver eines Mitbewerbers (Probe C). Bei dem aufbereiteten Zinkoxidpulver handelt es sich um das Produkt HiZox® von Animine.

| | |
|---|---|
| Auftrag: | Charakterisierung von Zinkoxidpulver |
| Durchführung: | DIL (Deutsches Institut für Lebensmitteltechnik E.V.) |
| | Anschrift: Professor-von-Klitzing-Straße 7, 49610 Quakenbrück |
| Probeneingang: | 22.06.2017 (ungekühlt) |

Probenkennzeichnung / angewendete Methoden:

| Probenbezeichnung | SV (PGV) | SV (BET) | REM | DIL-Nr. |
|---|---|---|---|---|
| Probe A | x | x | x | 17/571 |
| Probe B | x | x | x | 17/572 |
| Probe C | x | x | x | 17/573 |

### Genaue exakte Proben-Art:

Probe A: Zinkoxidpulver feed grade, Standardware Fa. Grillo
Probe B: Zinkoxidpulver mikronisiert in einer Exzenterschwingmühle, ISF GmbH
Probe C: Zinkoxidpulver aufbereitet, Vergleichsprodukt eines Mitbewerbers

### Methoden:

***Partikelgrößenverteilung(PGV):*** Laserbeugung 20nm-2mm; ISO 13320-1 ***Spez. Oberfläche(BET):*** Mehrpunkt-BET-Analyse (mind. 5 Punkt) im Bereich p/p0 = 0,05-0,3 gemäß ISO 9277:2010; Bestimmung der Adsorptionsisotherme im Bereich p/p0 = 0,05-0,30 mittels Stickstoff bei 77 K

### Raster-Elektronenmikroskopie(REM):

Den Pulverproben wurden Stichproben entnommen, auf selbstklebenden Kohleträgern fixiert und in der Präparationsanlage mit Gold besputtert.
Die Untersuchung im Raster-Elektronenmikroskop (REM) erfolgt bei Raumtemperatur.

Im REM wird ein Elektronenstrahl erzeugt, der mit einer Spannung von 1 bis 30 kV beschleunigt wird. Dieser Primärelektronenstrahl wird in einem isometrischen Muster auf die Probenoberfläche gelenkt. Das Sekundärelektronensignal, das durch den Primärstrahl erzeugt wird, ändert sich mit der Beschaffenheit der Probenoberfläche und dem Anstellwinkel des Strahles. Deshalb entsteht das Bild aus einer Reihe von Lichtflecken deren Helligkeit sich ändert. Da die Sekundärelektronen durch die magnetischen Linsen gezwungen werden können, einer gekrümmten Bahn zu folgen, ist es möglich, Oberflächenprofile sichtbar zu machen, die außerhalb der direkten Ziellinie des Kollektors liegen. So ist eine große Tiefe des Feldes möglich und außerdem wird ein dreidimensionaler Effekt erzeugt, weil Merkmale, die näher am Kollektor liegen, heller erscheinen, als jene, die weiter entfernt sind. Das entstehende Bild wird elektronisch gespeichert.

### Ergebnisse

Siehe auch nachfolgende Tabellen und Diagramme

| Probenbezeichnung | Spez.Oberfläche PGV (m²/g) | Spez.Oberfläche BET (m²/g) |
|---|---|---|
| Probe A | 0,078 | 0,34 |
| Probe B | 0,316 | 0,96 |
| Probe C | 0,550 | 44,91 |

### Raster- Elektronenmikroskopie:

Siehe nachfolgende Bilder

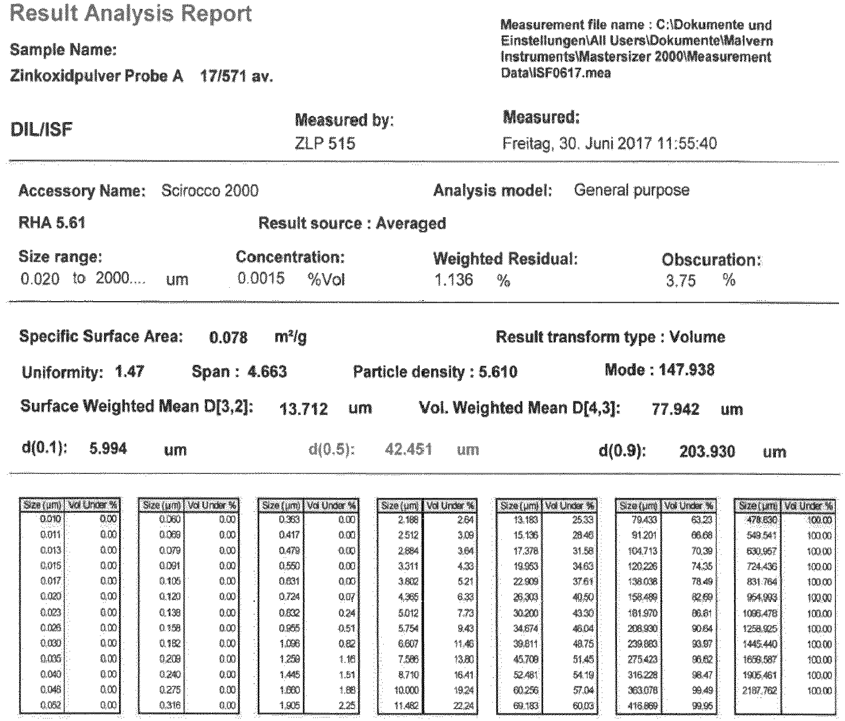

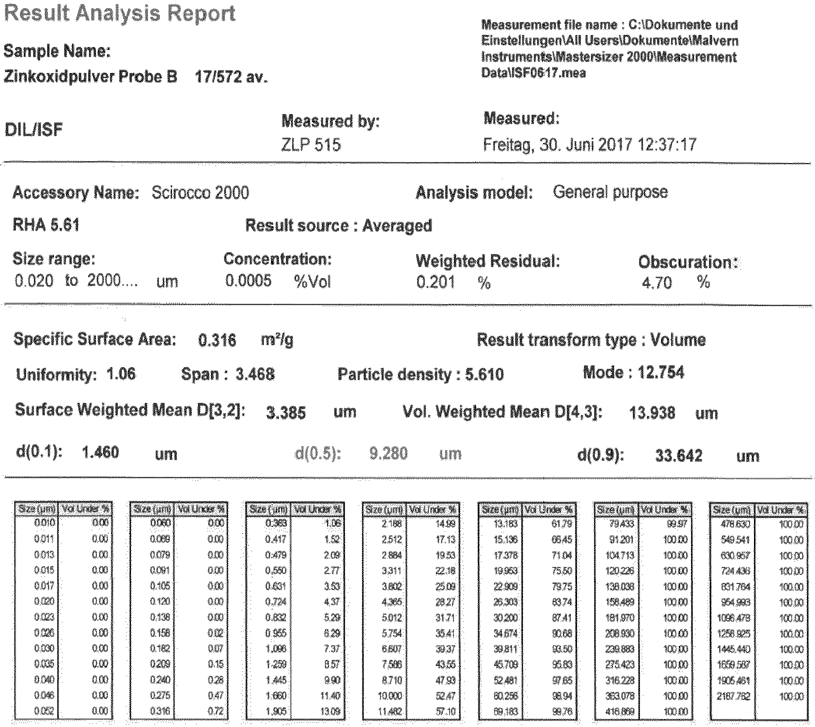

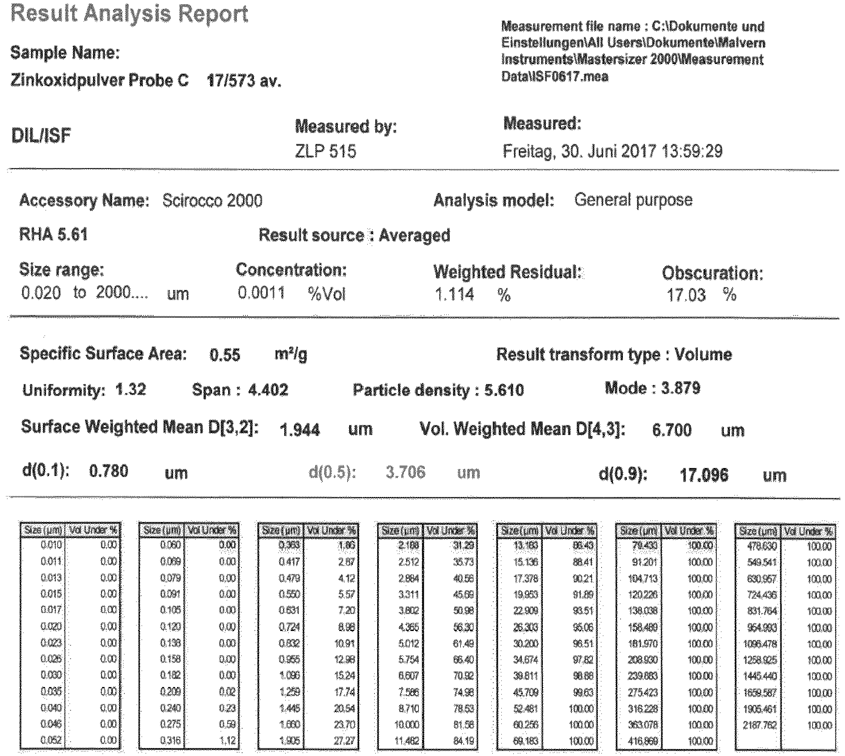

Gemäß der nachfolgenden Untersuchung betreffend Effekte auf pH-Wert und Löslichkeit durch Bearbeitung von ZnO in der Exzenter-Schwingmühle erhöht das erfindungsgemäße Zinkoxid im Vergleich zur Standardware den pH-Wert von destilliertem Wasser und löst sich stärker als die Standardware in destilliertem Wasser auf.

### Thema: Effekte auf pH-Wert und Löslichkeit durch Bearbeitung von ZnO in der Exzenterschwingmühle

### Hintergrund:

Es wurde festgestellt, dass sich bei einer unterschiedlich intensiven Bearbeitung von Zinkoxid in der Exzenterschwingmühle der pH-Wert einer wässrigen Suspension des Zinkoxids verändert. Es wird ein höherer pH-Wert gemessen, wenn das Zinkoxid in der Exzenterschwingmühle bearbeitet wurde.

### Probenmaterial Bezeichnung und Kennzeichnung:

**ZnO (102085) unbearbeitet:** Zinkoxidpulver feed grade, Standardware Fa. Grillo
**ZnO (102085) nach 10 Min.:** Zinkoxidpulver 10 Min mikronisiert in einer Exzenterschwingmühle, ISF GmbH
**ZnO (102085) nach 20 Min.:** Zinkoxidpulver 20 Min mikronisiert in einer Exzenterschwingmühle, ISF GmbH
**ZnO (102085) nach 30 Min.:** Zinkoxidpulver 30 Min mikronisiert in einer Exzenterschwingmühle, ISF GmbH
**ZnO (102085) nach 60 Min.:** Zinkoxidpulver 60 Min mikronisiert in einer Exzenterschwingmühle, ISF GmbH
**ZnO Mitbewerber:** Zinkoxidpulver aufbereitet, Vergleichsprodukt eines Mitbewerbers

### Messungen und Ergebnisse:

Das Probenmaterial, welches unterschiedliche Zeiten in der Exzenterschwingmühle bearbeitet wurde, wurde jeweils in eine 250 ml Schottflasche eingewogen (1 g oder 10 g) und mit 100 g aqua dest versetzt. In gleicher Art wurde die Probe eines Mitbewerbers, der eine besonders hohe Aktivität seines Zinkoxid-Materials auslobt, untersucht.

Es wurde für exakt 1 h intensiv geschüttelt und anschließend für 30 min ruhend stehen gelassen, damit sich das Probenmaterial absetzen konnte. Der Überstand wurde vorsichtig in ein 100 ml Becherglas überführt und es folgte die Messung des pH-Wertes direkt im flüssigen Überstand. Die Ergebnisse dazu sind in Tabelle 1: "*pH-Werte in wässrigen Lösungen von verschiedenen Zinkoxid-Proben*" dargestellt.

**Tabelle 1: pH-Werte in wässrigen Lösungen von verschiedenen Zinkoxid-Proben**

| | ZnO (102085) unbearbeitet | ZnO (102085) nach 10 Min. | ZnO (102085) nach 20 Min. | ZnO (102085) nach 30 Min. | ZnO (102085) nach 60 Min. | ZnO Mitbewerber |
|---|---|---|---|---|---|---|
| 1 g + 100 ml aqua dest | 7,34 | 7,67 | 7,73 | 7,73 | 7,75 | 8,27 |
| 10 g + 100 ml aqua dest | 8,01 | 8,62 | 8,69 | 8,70 | 8,85 | 8,60 |

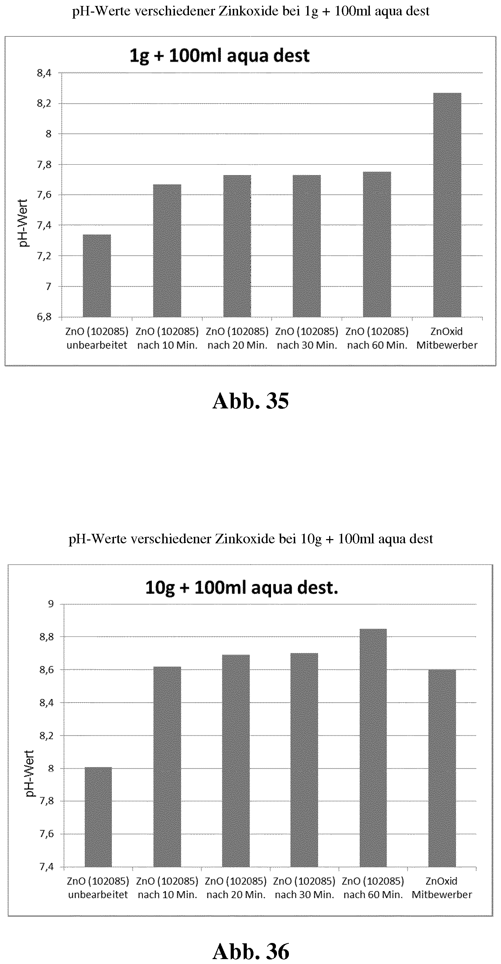

Eine graphische Darstellung der Ergebnisse erfolgt in Abb. 35: "*pH-Werte verschiedener Zinkoxide bei 1 g* + *100 ml aqua dest*" sowie in Abb. 36: "*pH-Werte verschiedener Zinkoxide bei 10 g* + *100 ml aqua dest*"

Zusätzlich wurde in einigen Proben des Ansatzes "1 g Zinkoxid + 100 ml aqua dest" noch der gelöste Anteil an Zink gemessen und in folgender Tabelle dargestellt.

| | ZnO (102085) unbearbeitet | ZnO (102085) nach 20 Min. | ZnO (102085) nach 60 Min. | ZnO Mitbewerber |
|---|---|---|---|---|
| Gelöstes Zink bei 20 °C | 2,2 mg/l | 5,8 mg/l | 7,4 mg/l | 3,0 mg/l |

### Deutung der Ergebnisse:

Oxide von unedlen Metallen, können mit Wasser zu Basen reagieren und Laugen bilden. Die Reaktion läuft für zweiwertige Metalle nach folgendem allgemeinen Schema ab, wobei Me allgemein für ein Metall-Element steht, z. B. Calcium, Magnesium oder Zink:

MeO + H₂O → Me(OH)₂ → Me²⁺ + 2 OH⁻

*(*Römpp Lexikon der Chemie, 10. vollständig überarbeitete Auflage, Georg Thieme Verlag, Band 4, Seite 3071*)*

Der pH-Wert einer wässrigen Suspension solcher Metalloxide liegt dann im alkalischen Bereich, da die Hydroxid-Ionen (OH⁻) die Oxoniom-Ionen (H₃O⁺) neutralisieren, und somit die Oxonium-Konzentration abnimmt sowie der pH-Wert ansteigt.

### Beispiel:

Der Anstieg des pH-Wertes von 7,34 auf 7,73 bedeutet, dass sich die H₃O⁺-Ionen Konzentration von 10^{-7,34} (bzw. 4,571E-08) mol/l auf 10^{-7,73} (bzw. 1,762E-08) mol/l verringert hat, also um rund 62 %.

In den durchgeführten Messungen konnte festgestellt werden, dass sich der pH-Wert in den Lösungen mit dem in der Exzenterschwingmühle bearbeiteten Zinkoxid deutlich erhöhte. Normalerweise wird Zinkoxid als praktisch unlösliches Metalloxid betrachtet, denn seine Löslichkeit liegt lediglich bei 1,6 mg/l Wasser (GESTIS-Stoffdatenbank des IFA abgerufen 16. Februar 2017). Die deutliche Erhöhung des pH-Wertes und der höhere Anteil an gelösten Zink-Ionen erklärt sich über eine verstärkte Reaktion des Zinkoxids mit Wasser, was wiederum eine insgesamt verstärkte Reaktivität des Zinkoxids bedeutet.

Mit steigender Dauer der Aktivierung in der Exzenterschwingmühle kann ein weiterer Anstieg des pH-Wertes sowie eine Erhöhung des Anteils an gelösten Zink-Ionen und somit auch eine Steigerung der Reaktivität mit der Mahldauer des Zinkoxids beobachtet werden. So kann man auch anhand dieses Verlaufs die Wirkung der Exzenterschwingmühle auf das Zinkoxid ableiten.

Das ebenfalls untersuchte Zinkoxid eines Mitbewerbers zeigte beim pH-Wert ähnlich hohe Werte wie das in der Exzenterschwingmühle aktivierte Zinkoxid, lag jedoch bei dem Anteil des gelösten Zinks im Bereich des normalen, unbehandelten Zinkoxids.

Gemäß der nachfolgenden Untersuchung hat das erfindungsgemäße Zinkoxid im Vergleich zur Standardware Vorteile in den ersten Wochen der Ferkelaufzucht hinsichtlich der Entwicklung der Lebendmasse und der Durchfallhäufigkeit.

### Zur Wirkung von aktiviertem Zinkoxid auf die Darmgesundheit und Leistung von Aufzuchtferkeln

### Allgemein

Spurenelemente - Zink, Kupfer, Mangan, Selen, Kobalt, Eisen, Jod etc. - sind lebensnotwendige Nährstoffe für alle Tiere. Sie haben viele Funktionen im Körper und sind somit einzeln oder gemeinsam im allgemeinen Stoffwechsel, der Reproduktion, dem Immunsystem, dem Wachstum, der Entwicklung und Regenerierung verschiedener Gewebe usw. beteiligt.
Zink ist wichtiger Bestandteil vieler Enzyme (>300), die an der Synthese und dem Abbau von Kohlenhydraten, Fetten, Proteinen, Nukleinsäure als auch dem Stoffwechsel anderer Mikronährstoffe beteiligt sind. Es stabilisiert die molekulare Struktur von zellulären Bestandteilen und Membranen und trägt auf diese Weise zur Erhaltung der Zell- und Organintegrität bei. Des Weiteren spielt Zink eine wichtige Rolle in der Transkription und somit Genexpression. Durch seine Beteiligung an diesen fundamentalen Prozessen ist Zink essentiell für alle Lebensformen. Ferner hat Zink eine zentrale Funktion im Immunsystem, da es sowohl das zelluläre als auch humorale Immunsystem beeinflusst.

### Darmstabilisierende Wirkung

Ferner ist Zinkoxid in vielen Ländern der EU ein weitverbreiteter pharmazeutischer Zusatz im Futter von frühabgesetzten Ferkeln, um Durchfälle zu vermeiden. Hier wird Zinkoxid in therapeutischen Dosierungen weit über den futtermittelrechtlichen Grenzwerten (3000 vs. 150 ppm) für max. 14 Tage nach dem Absetzen eingesetzt, wenn es durch einen Tierarzt verschrieben wurde. Wohingegen in einigen Ländern, wie z. B. auch der BRD, ein Verbot für solche Anwendungen vorliegt. Durch den erheblichen Umwelteinfluss hoher ZnO-Dosierungen wird dieser Anwendungsbereich aber sehr kritisch in der EU betrachtet.

### Zinkoxid - Wirkungsweise

Der Mechanismus hinter den günstigen Einflüssen von hohen Zinkoxiddosierungen zur Vermeidung von Durchfall und der Förderung der Leistung von abgesetzten Ferkeln kann noch nicht vollständig erklärt werden. Denn nur ein geringer Anteil des verabreichten ZnO wird im Verdauungstrakt absorbiert, wohingegen der größte Teil über den Darm wieder ausgeschieden wird. Nachfolgende Ansätze erklären die möglichen, vielfältigen, regulativen Einflüsse von ZnO auf die Leistung und Darmgesundheit von Ferkeln.
- Eine gute Versorgung des Organismus mit Zink hat einen leistungsfördernden Effekt. Die Zinkabsorption ist nach dem Absetzen der Ferkel auf einem stark verminderten Niveau, sodass sich die Bioverfügbarkeit auch entsprechend reduziert. Hohe Gehalte an ZnO im Futter erhöhen den Zinkplasmaspiegel parallel mit gesteigerter Futteraufnahme.
- ZnO reguliert die Flora des Verdauungstrakts. Hohe Gehalte an Zn können die Darmflora nach einem Futterwechsel stabilisieren und die Anheftung von E. coli an der Darmwand hemmen.
- ZnO verbessert die Struktur und Funktion des Darmgewebes und vermindert somit das Auftreten von Darmschädigungen in kritischen Phasen durch eine begünstigte Regeneration der Zellen und Stimulation des Gewebewachstums
- ZnO reduziert die Sekretion von Ionen in das Darmlumen, was dazu beiträgt, Durchfallerscheinungen zu vermindern.
- ZnO reduziert die Freisetzung von Histamin mit dessen entzündungsfördernden und immunhemmenden Eigenschaften.
- ZnO reguliert die Sekretion von Darm-Hirn-Peptiden, die die Futteraufnahme auf Ebene des Zentralen Nervensystems stimulieren.

### Versuchsgestaltung

Die ISF führte einen Fütterungsversuch mit Absetzferkeln im Zeitraum vom 22.03. bis 05.04.2017 in der Stallanlage von Gut Hülsenberg durch.

In diesem Fütterungsversuch sollte der Zusatz von aktiviertem Zinkoxid im Ferkelaufzuchtfutter geprüft werden. Die nachfolgenden Zulagen wurden über die gesamte Versuchsperiode dem Alleinfutter zugesetzt.

Kontrollgruppe: Alleinfutter + Zusatz von 160 ppm Zinkoxid (Standardware) Versuchsgruppe: Alleinfutter + Zusatz von 160 ppm aktivierten Zinkoxid
Als Versuchsparameter wurden die Futteraufnahme, die Lebendmassezunahme, der Futteraufwand sowie eine Kotbonitierung herangezogen.

### Material und Methoden

### Versuchsvorbereitung

Es wurden 200 Absetzferkel (DanAvl x Pietrain) mit einem mittleren Lebensalter von 26 Tagen, randomisiert nach der Lebendmasse und dem Geschlecht, über acht Buchten eines Flatdeckabteils aufgestallt. Alle Tiere und Buchten erhielten individuelle Kennzeichnungen, um eine tier- und buchtenspezifische Datenerfassung zu gewährleisten.

### Versuchsperiode

Die Ferkel wurden nach einer vierwöchigen Säugezeit von den Muttersauen abgesetzt. Der Fütterungsversuch begann direkt nach der Anlieferung der Ferkel und wurde über 14 Tage durchgeführt.

### Futter und Fütterung

Die Fütterung der Ferkel erfolgte über 14 Tage mit einem Absetzfutter ohne Zusatz von Zink. Durch die Verwendung von Vormischungen (Alleinfutter + Zusatzstoff) wurden die entsprechenden Mengen an Zinkoxid mittels Additivdosierer dem Alleinfutter zugesetzt.

In den ersten 7 Tagen nach Anlieferung wurden die Ferkel dreimal täglich rationiert (ca. 80 % der Futterkurve) über portable Metalltröge und die Futterschalen der Breiautomaten gefüttert. Das Futter wurde zur besseren Aufnahme mit warmem Wasser angefeuchtet. Am sechsten und siebenten Tag begann parallel die Fütterung über die Breiautomaten. Anschließend erfolgte bis zum Ende der Versuchsperiode die ad libitum Fütterung ausschließlich über die Breiautomaten mit Vorratsbehältern.

### Gesundheitsstatus

Der wichtigste Aspekt des Versuches war der Einfluss von aktiviertem Zinkoxid auf die Darmgesundheit der Aufzuchtferkel. Hier war die Häufigkeit von Durchfällen innerhalb des Versuchszeitraums ein wesentliches Indiz für die Wirksamkeit des Zusatzstoffes. Aus dem genannten Grund wurden keine prophylaktischen Maßnahmen zur Darmstabilisierung durchgeführt.
Um die Durchfallhäufigkeit zu bewerten, wurde die Anzahl an Ferkeln mit Durchfall pro Bucht täglich ermittelt.

Bewertung der Kotkonsistenz:
1 Normal: TM > 25 %
2 Wässrig (Durchfall): TM < 18 %

### Ergebnisse

### Darmstabilität

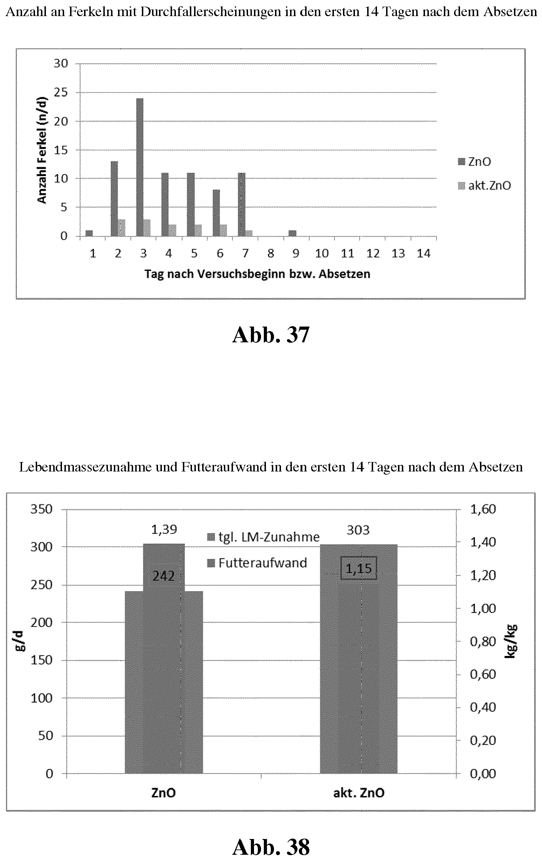

Innerhalb der ersten neun Tage nach Versuchsbeginn bzw. dem Absetzen der Ferkel sind bei Einzeltieren beider Gruppen Durchfallerscheinungen aufgetreten. Wie in Abbildung 37 dargestellt, wies die Behandlungsgruppe mit aktiviertem ZnO eine erheblich geringere Anzahl an Tieren mit Durchfall auf. So konnte z. B. am dritten Versuchstag nur bei drei Ferkeln der Behandlungsgruppe wässriger Kot festgestellt werden, wohingegen bei 24 Tieren der Kontrollgruppe (Standard-ZnO) diese Kotveränderung auftrat.

### Lebendmassezunahme und Futteraufwand

Auch die zootechnischen Leistungen der Ferkel wiesen innerhalb des Versuchszeitraums eindeutige Unterschiede auf. Wie in Abbildung 38 dargestellt, war die tägliche Lebendmassezunahme der Ferkel, die aktiviertes ZnO erhielten, um ca. 25 % höher und der Futteraufwand wiederum um ca. 20 % geringer als in der Kontrollgruppe mit Standard-ZnO, ohne gravierende Unterschiede in der Futteraufnahme aufzuzeigen. Auch diese Ergebnisse bestätigen eine verbesserte Stabilität des Verdauungssystems der Aufzuchtferkel durch den Zusatz von aktiviertem ZnO im Futter.

### Fazit

Aktiviertes Zinkoxid zeigte bei einer Dosierung von 160 ppm im Futter einen eindeutigen Effekt auf die Stabilisierung des Verdauungssystems von Aufzuchtferkeln. Innerhalb des gewählten Versuchsraums, der kritischen Phase nach dem Absetzen, wurde die Durchfallhäufigkeit vermindert als auch die zootechnische Leistung der Ferkel signifikant verbessert.

## Patentansprüche

1. Verfahren zum Herstellen von Zinkoxid zur alimentären Verwendung bei Schweinen, anderen monogastrischen Tieren, Pferden oder Wiederkäuern, bei dem Zinkoxid im festen Aggregatzustand auf einen mittleren Durchmesser der Partikel von maximal 50 µm vermahlen und zugleich mechanisch aktiviert wird.

2. Verfahren nach Anspruch 1, bei dem das Ausgangsprodukt ein Zinkoxidpulver mit einem mittleren Durchmesser der Partikel von über 50 µm ist.

3. Verfahren nach Anspruch 1 oder 2, bei dem das Zinkoxid in einer Mühle für die Feinstmahlung in einer Mühle für die Mikrofeinmahlung oder in einer Mühle für die Nanofeinmahlung vermahlen und mechanisch aktiviert wird.

4. Verfahren nach einem der Ansprüche 1 bis 3, bei dem das Zinkoxid mittels einer Kugelmühle, Schwingmühle, Planetenmühle, Rührwerkmühle oder einer anderen Mahlkörpermühle vermahlen und mechanisch aktiviert wird.

5. Verfahren nach Anspruch 4, bei dem das Zinkoxid mittels einer Exzenter-Schwingmühle vermahlen und mechanisch aktiviert wird.

6. Verfahren nach einem der Ansprüche 1 bis 5, bei dem das Zinkoxid im trockenen Zustand vermahlen und mechanisch aktiviert wird.

7. Verfahren nach einem der Ansprüche 1 bis 6, bei dem das Zinkoxid auf einen mittleren Partikeldurchmesser von maximal 30 µm, vorzugsweise von maximal 20 µm, vorzugsweise von maximal 15 µm, vorzugsweise von maximal 10 µm vermahlen wird.

8. Verfahren nach einem der Ansprüche 1 bis 7, bei dem das Zinkoxid auf einen mittleren Partikeldurchmesser von mindestens 2 µm, vorzugsweise von mindestens 5 µm vermahlen wird.

9. Verfahren nach einem der Ansprüche 1 bis 8, bei dem das Zinkoxid so vermahlen wird, dass seine spezifische Oberfläche mindestens 0,2 m²/g, vorzugsweise mindestens 0,25 m²/g beträgt.

10. Verfahren nach einem der Ansprüche 1 bis 9, bei dem das Zinkoxid so vermahlen wird, dass seine spezifische Oberfläche maximal 0,5 m²/g, vorzugsweise maximal 0,45 m²/g beträgt.

11. Verfahren nach einem der Ansprüche 1 bis 10, bei dem das Zinkoxid während einer Mahldauer von bis zu 10 Minuten, vorzugsweise von bis zu 20 Minuten, vorzugsweise von bis zu 30 Minuten vermahlen und mechanisch aktiviert wird.

12. Verfahren nach einem der Ansprüche 1 bis 11, bei dem das Zinkoxid so vermahlen und mechanisch aktiviert wird, dass die Sättigungskonzentration der Zinkionen in Wasser mindestens 4 mg/l, vorzugsweise mindestens 5 mg/l beträgt.

13. Verfahren gemäß einem der Ansprüche 1 bis 12, bei dem das Zinkoxid so vermahlen und mechanisch aktiviert wird, dass der pH-Wert nach Zugabe von 10 g Zinkoxid zu 100 ml destilliertem Wasser, intensivem Schütteln über eine Stunde und Ruhenlassen über 30 Minuten im flüssigen Überstand mindestens 8,6, vorzugsweise mindestens 8,8 beträgt.

14. Zinkoxid zur alimentären Verwendung bei Schweinen, anderen monogastrischen Tieren, Pferden oder Wiederkäuern, erhältlich durch das Verfahren nach einem der Ansprüche 1 bis 13.

15. Zinkoxid zur alimentären Verwendung in einem prophylaktischen oder therapeutischen Verfahren bei Schweinen, insbesondere zur vorbeugenden alimentären Verwendung bei durchfallgefährdeten Schweinen, insbesondere Absetzferkeln, oder bei anderen monogastrischen Tieren, erhältlich durch das Verfahren nach einem der Ansprüche 1 bis 13.

16. Futtermittel zur prophylaktischen oder therapeutischen Verwendung bei Schweinen, insbesondere zur vorbeugenden alimentären Verwendung bei durchfallgefährdeten Schweinen, insbesondere Absetzferkeln, oder anderen monogastrischen Tieren , enthaltend maximal 3.000 ppm, vorzugsweise maximal 2.000 ppm, vorzugsweise maximal 1.000 ppm, vorzugsweise maximal 160 ppm, vorzugsweise maximal 120 ppm, vorzugsweise maximal 100 ppm, vorzugsweise maximal 65 ppm Zinkoxid, erhältlich durch das Verfahren nach einem der Ansprüche 1 bis 13.

17. Ergänzungsfuttermittel zur Unterstützung der Regenerierung von Hufen, Füßen und Haut bei Schweinen, Pferden oder Wiederkäuern, das Zinkoxid enthält, das erhältlich ist durch das Verfahren nach einem der Ansprüche 1 bis 13.

18. Verwendung von Zinkoxid erhältlich durch das Verfahren nach einem der Ansprüche 1 bis 13 bei der Ernährung von Schweinen, Pferden oder Wiederkäuern zur Unterstützung der Regenerierung von Hufen, Füßen und Haut.
